# EUROPEAN PATENT APPLICATION

(11) **EP 3 480 786 A1**
(43) Date of publication of application: **08.05.2019**
(21) Application number: 18203324.1
(22) Date of filing: 30.10.2018
(51) Int. Cl.: G06T 7/00, G06T 7/11

(54) **MEDICAL IMAGE OBJECT DETECTION WITH DENSE FEATURE PYRAMID NETWORK ARCHITECTURE IN MACHINE LEARNING**

(30) Priority: 03.11.2017 US 201715802893
(71) Applicant: Siemens Healthcare GmbH, 91052 Erlangen (DE)
(72) Inventor: Georgescu, Bogdan, Plainsboro, NJ New Jersey 08536 (US); Liu, Siqi, Princeton, New Jersey 08540 (US); Xu, Daguang, Princeton, NJ New Jersey 08540 (US); Comaniciu, Dorin, Princeton Junction, NJ New Jersey 08550 (US); Zhou, Shaohua Kevin, Princeton, NJ 08540 (US); Wengrowski, Eric, Somerset, 08873 (US)
(74) Representative: Patentanwälte Bals & Vogel

(57) **Abstract**

For object detection (56), deep learning (44) is applied with an architecture designed for low contrast objects, such as lymph nodes. The architecture uses a combination of dense deep learning or features, which employs feed-forward connections between convolutions layers (22), and a pyramidal arrangement of the dense deep learning using different resolutions.

## Description

### Background

The present embodiments relate to object detection and machine learning of the object detection, such as lymph nodes.

Lymph nodes are routinely examined in all types of cancer treatment, including lymphoma. Size is commonly measured throughout radiation or chemotherapy to monitor the effectiveness of cancer treatment. Physicians assess lymph node size or characteristic in patients using three-dimensional (3D) computed tomography (CT) scans. This manual detection and measurement of lymph nodes from 3D CT images is cumbersome and error prone.

For automatic detection, deep learning is commonly used for organ and liver segmentation. For certain automatic medical image analysis tasks, computer-aided detection methods may achieve high sensitivities, but typically suffer from high false positives (FP) per patient. To solve this problem, a two-stage coarse-to-fine approach may be employed. U-Net is a neural network that uses available annotated samples more efficiently. The architecture consists of a contracting path to capture context and a symmetric expanding path that enables end-to-end learning from fewer images. This neural network for dense volumetric segmentation learns from sparsely annotated volumetric images. Successful training of deep networks often requires many thousand annotated training samples, which may not be available.

For automatic detection of lymph nodes, filtering using gradient, Haar, or convolutional networks have been applied. The convolutional networks use deep learning. Even with deep learning, automatic detection is challenging because lymph nodes have an attenuation coefficient similar to muscles and vessels and therefore low contrast to surrounding structures. Automatic lymph node detection is nevertheless desirable so physicians may treat patients more quickly and easily. However, there exists a significant gap in detection accuracy between previous automatic methods and the manual detection accuracy expected from a human.

### Summary

Systems, methods, and computer readable media are provided for object detection. Deep learning is applied with an architecture designed for low contrast objects, such as lymph nodes. The architecture uses a combination of dense deep learning, which employs feed-forward connections between convolutions layers, and a pyramidal arrangement of the dense deep learning using different resolutions.

In a first aspect of the invention, a method is provided for lymph node detection with a medical imaging system. A medical image of a patient is received. A machine-learnt detector detects a lymph node represented in the medical image. The machine-learnt detector includes a dense feature pyramid neural network of a plurality of groups of densely connected units where the groups are arranged with a first set of the groups connected in sequence with down sampling and a second set of the groups connected in sequence with up sampling and where groups of the first set connect with groups of the second set having a same resolution. The medical imaging system outputs the detection of the lymph node.
Preferred is a method, wherein the medical imaging system comprises a computed tomography (CT) system, and wherein receiving the medical image comprises receiving CT data representing a volume of the patient.
Preferred is further a method, wherein detecting by the machine-learnt detector comprises detecting with a fully convolutional network.
Preferred is a method, wherein detecting comprises detecting with the dense feature pyramid neural network comprising an initial convolutional layer down sampling the medical image.
Preferred is further a method, wherein detecting comprises detecting with each of the groups comprising a sequence of layers where each layer of the sequence concatenates output features from all previous ones of the layers in the sequence.
Preferred is a method, wherein detecting comprises detecting with the groups of the first set are in the sequence having the down sampling between each group of the first set, each group of the first set having different resolution than the other groups of the first set.
Further, a method is preferred, wherein detecting comprises detecting with the groups of the second set are in the sequence having the up sampling between each group of the second set, each group of the second set having different resolution than the other groups of the second set.
Preferred is a method, wherein detecting comprises detecting with the first set comprising an encoder and with the second set comprise a decoder.
Further, a method is preferred, wherein detecting comprises detecting with the dense feature pyramid neural network configured to output a heatmap at a resolution of the medical image.
Preferred is further a method, wherein detecting by the machine-learnt detector comprises applying a threshold to an output responsive to input of the medical image to the dense feature pyramid neural network. Detecting by the machine-learnt detector can further comprise performing non-maximal suppression to results of the applying of the threshold.
Preferred is a method, wherein outputting comprises generating a representation of the medical image with an annotation for the lymph node. Preferred is further a method, wherein detecting by the machine-learnt detector comprises detecting by the machine-learnt detector trained based on Gaussian blobs as segmentation of lymph nodes in training data, and wherein detecting comprises outputting Gaussian blobs for the medical image.

In a second aspect, a medical imaging system is provided for object detection. A medical scanner is configured to scan a three-dimensional region of a patient. An image processor is configured to apply a machine-learnt detector to data from the scan. The machine-learnt detector has an architecture including modules of densely connected convolutional blocks, up sampling layers between some of the modules, and down sampling layers some of the modules. The machine-learnt detector is configured to output a location of the object as represented in the data from the scan. A display is configured to display a medical image with an annotation of the object at the location based on the output.
Preferred is a medical imaging system, wherein the medical scanner comprises a computed tomography system, and wherein the image processor and the display are part of the computed tomography system. Further, a medical imaging system is preferred, wherein the medical scanner comprises a computed tomography system, and wherein the image processor and the display are part of the computed tomography system.
Preferred is a medical imaging system, wherein the architecture of the machine-learnt detector comprises a first set of the modules arranged in sequence with one of the down sampling layers between each of the modules of the first set and a second set of the modules arranged in sequence with one of the up sampling layers between each of the modules of the second set.
Further, a medical imaging system is preferred, wherein the machine-learnt detector is trained with Gaussian blobs as annotations in the training data and wherein the architecture outputs a heatmap.

In a third aspect, a method is provided for training for object detection. A neural network arrangement of sets of convolutional blocks is defined. The blocks in each set have feed-forward skip connections between the blocks of the set. The arrangement includes a down sampling layer between a first two of the sets and an up sampling layer between a second two of the sets. A machine trains the neural network arrangement with training data having ground truth segmentation of the object. The neural network as trained is stored.
Preferred is a method according to the third aspect of the invention, wherein defining comprises defining with connections between the first two and the second two of the sets with a same resolution.
Preferred is further a method according to the third aspect of the invention, wherein training comprises training (44) with the ground truth segmentation comprising Gaussian blobs.
The steps of the method according to the third aspect of the invention can be combined with the steps of the method according to the first aspect of the invention and vice versa.

Any one or more of the aspects described above may be used alone or in combination. These and other aspects, features and advantages will become apparent from the following detailed description of preferred embodiments, which is to be read in connection with the accompanying drawings. The present invention is defined by the following claims, and nothing in this section should be taken as a limitation on those claims. Further aspects and advantages of the invention are discussed below in conjunction with the preferred embodiments and may be later claimed independently or in combination.

### Brief Description of the Drawings

The components and the figures are not necessarily to scale, emphasis instead being placed upon illustrating the principles of the embodiments. Moreover, in the figures, like reference numerals designate corresponding parts throughout the different views.
Figure 1 is a flow chart diagram of one embodiment of a method for object detection training;
Figure 2 illustrates an example neural network architecture using modules of densely connected convolutional blocks with encoder down sampling between some modules and decoder up sampling between other modules;
Figure 3 is a flow chart diagram of one embodiment of a method for object detection by application of a trained dense feature pyramid neural network;
Figure 4 illustrates an example image showing Gaussian blobs and corresponding detected centers;
Figure 5 shows predicted and actual positive and negative detection of lymph nodes using a dense feature pyramid neural network trained with Gaussian blobs;
Figure 6 shows predicted and actual positive and negative detection of lymph nodes using a dense feature pyramid neural network trained with fully annotated segmentation masks; and
Figure 7 is a block diagram of one embodiment of a system for object detection.

### Detailed Description of Embodiments

Automatic lymph node detection is challenging due to clutter, low contrast, and variation in shape and location of the lymph nodes. Lymph nodes occur adjacent different types of tissue throughout the body. Lymph nodes may be commonly confused with other structures.

Lymph node detection uses a dense feature pyramid network. A trained convolutional neural network provides automatic lymph node detection in CT data. Densely connected blocks in modules are used in an encoder-decoder pyramid architecture, allowing efficient training from fewer images. A densely connected convolutional neural network architecture is used in one or more of the modules. Densely connected neural networks have recently emerged as the new state-of-the-art architecture for object recognition tasks. Feed-forward connections between all layers in the module are used where the feature-maps of all preceding layers are used as inputs into all subsequent layers. This allows for substantially deeper neural network architectures that contain fewer parameters, alleviating vanishing-gradient problems, strengthening feature propagation, encouraging feature reuse, and drastically reduces over-fitting in training. This results in better performance, fastertraining times, and reduced memory use.

The dense feature pyramid network deals well with low contrast, small object detection with variation in background. The dense feature pyramid network achieves significant improvement over previous deep learning-based lymph node detection. Even trained using only 645 patient scans, 98.1% precision and 98.1% recall on validation data is achieved with 1 false positive for every 6 patients. This is an improvement over 85% recall with 3 false positives per patient of Shin, et al. in "Deep convolutional neural networks for computer-aided detection: Cnn architectures, dataset characteristics and transfer learning," IEEE transactions on medical imaging, vol. 35, no. 5, pp 1285-1298, 2016.

Other objects in the body of a patient may be detected. Lymph node examples are used herein. Other objects include lesions, such as liver tumors, kidney tumors, lung nodules, or breast cysts. The machine-learnt detector is trained to detect any type of object.

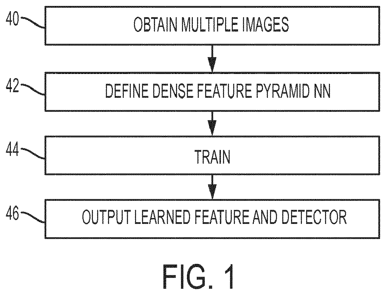

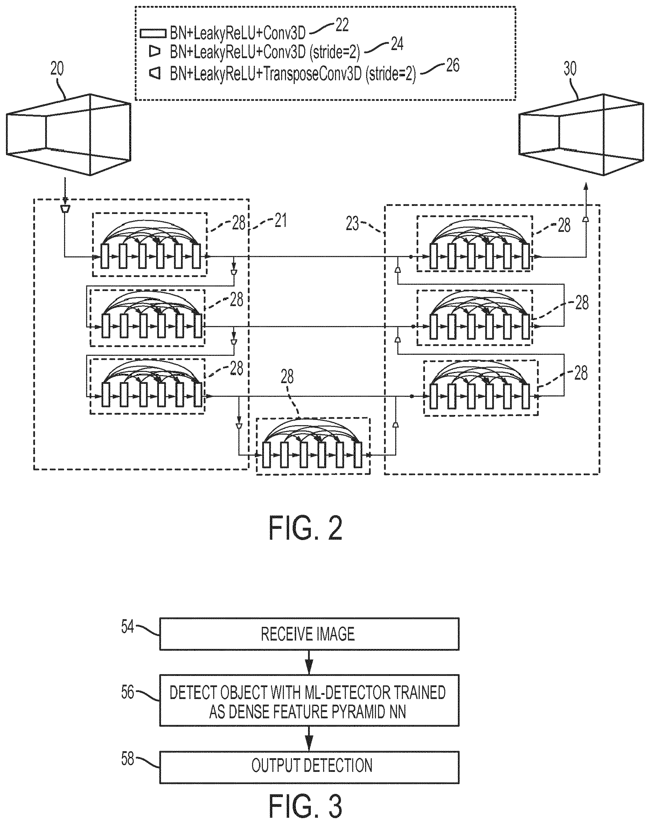

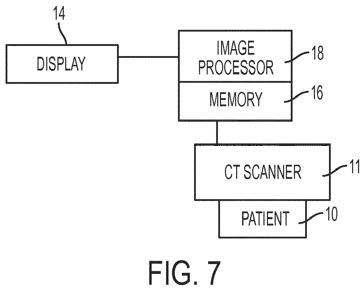

Figures 1 and 3 show methods for object detection. The method for object detection may be a method to learn how to detect the object or may be a method for detecting the object. Figure 1 is directed to machine training of the object detector. Figure 3 is directed to application of a machine-learnt object detector. In both cases, a machine, such as an image processor, computer, or server, implements some or all the acts. The same or different machine is used for training and application. The system of Figure 7 implements the methods in one embodiment.

A user may select the image files for application of the object detector by the processor or select the images from which to learn features and a classifier by a processor. Use of the machine allows processing large volumes (e.g., images of many pixels and/or many images) of information that may not be efficiently handled by humans, may be unrealistically handled by humans in the needed time frame, or may not even be possible by humans due to subtleties and/or timing. The machine may learn in a way different than a human to recognize the object in a way different than a human. Use of the architecture discussed herein may make the machine operate more quickly, use less memory, and/or provide better results in application and/or training than other automated approaches.

The methods are provided in the orders shown, but other orders may be provided. For Figure 1, acts 42 and 44 may be performed as one act.

Additional, different or fewer acts may be provided. For example, act 46 of Figure 1 is not provided. As another example, act 58 of Figure 3 is not provided. In yet other examples, acts for capturing images and/or acts using detected information are provided.

Figure 1 shows a method for object detection through learning by an image processor. The deep dense pyramid architecture used for training provides for accurate detection of the object.

In act 40, images of a same type of object (e.g., lymph node) are obtained. The images are obtained by data transfer, capture, and/or loading from memory. Any number of pictures of a same type of object is obtained, such as one, two, tens or hundreds of images of the object. The images are obtained with a same scanner or different scanners. The object as occurring in many different patients is included in the images. Where the object occurs with different backgrounds, the images are of the object in the various backgrounds.

The images are captured using any one or more scanners. For example, images of organs are captured using x-ray, computed tomography, fluoroscopy, angiography, magnetic resonance, ultrasound, positron emission tomography, or single photon emission computed tomography. Multiple images of the same or different patients using the same or different imaging modality (i.e., sensors or type of sensor) in the same or different settings (e.g., field of view) may be obtained. The object of interest in a medical image may be an organ (e.g., lymph node), a cyst, a tumor, calcification, or other anomaly or lesion.

The images represent volumes. Three-dimensional datasets are obtained. In alternative embodiments, two-dimensional datasets representing planes are obtained. The obtained images are data that may be used to generate an image on a display, such as a medical image being scan data from medical imaging. The obtained images are from data being processed to generate an image, data formatted for display, or data that has been used to display.

The medical images are used for training in act 44. The medical images may be used as received or may be pre-processed. In one embodiment of pre-processing, the received images are normalized. Since different settings, imaging systems, patients being scanned, and/or other variations in acquiring images may result in different offsets and/or dynamic ranges, normalization may result in more uniform representation of the object. Any normalization may be used, such as setting a maximum value to 1 with all other values linearly scaled between 0 and 1. Each volumetric scan or medical image is individually normalized.

To increase training efficiency, each of the medical images (e.g., patient scans) is randomly sampled. Rather than using each of the entire volume scans, the training data is randomly sampled. For example, a 32x32x32 window is used. Other sizes may be used. A center location of the window is defined, and the center is randomly placed relative to the medical image. Placement relative to the object to be detected may alternatively be used. The placement is repeated N times (e.g., N=200) for each instance of the object or patient scan. The result is N sets of 32x32x32 samples of the medical image per object and/or per patient scan. These 32x32x32 samples have random translations, and may or may not contain lymph nodes.

The training data includes a ground truth indication of the object. The ground truth indication is a segmentation of the object, such as a marker, trace, boarder, or other segmentation of a lymph node. The medical images, such as volumetric CT patient body scans, are physician-annotated. These volumetric CT scans have a 1.5 millimeter resolution in the (*x, y, z*)axis.

In one embodiment, the annotation designating the object is a Gaussian blob. Other distributions than Gaussian may be used. The blob generally marks the location of lymph node. The blob is centered around the centroid of each lymph node, scaled between 0 and 1, with the largest values found at the center of each blob. The blob is an expected size of the object, such as being larger than an average longest dimension of the lymph node by 25%, 50%, or other relative size. Alternatively, the radius of the blob is set to be the same as or smaller than the average radius of the object. In alternative embodiments, each blob is sized to the object over which the blob is placed. The blob may be warped or shaped to match in general without full segmentation or identification of the 3D border.

Volumetric data is abundant in biomedical imaging. Deep learning-based approaches often require myriad annotated data for training. Obtaining high-quality annotations of this data is difficult, since only 2D slices are shown on a computer screen. Annotating large volumes in a slice-by-slice manner is unreliable, tedious, and inefficient since neighboring slices show similar information. Full annotations (i.e., tracing the object boundary) of 3D volumes is not an effective way to create large and rich training data sets that would generalize well. Fully segmented annotations are substituted with Gaussian blobs centered on the targets. The blobs act as heat maps for each lymph node. This solution is more attractive than simply annotating with a single point for each lymph node because detecting the exact centroid of each target is less important than identifying the region or size. Further, the blob approach makes use of more spatial context and eases the training process. In alternative embodiments, a single point annotation or full segmentation (i.e., tracing) is used to designate the ground truth in the training data.

In act 42, a neural network (e.g., deep learning) arrangement is defined. The definition is by configuration or programming of the learning. The number of layers or units, type of learning, and other characteristics of the network are controlled by the programmer or user. In other embodiments, one or more aspects (e.g., number of nodes, number of layers or units, or type of learning) are defined and selected by the machine during the learning.

Deep architectures include convolutional neural network (CNN) or deep belief nets (DBN), but other deep networks may be used. CNN learns feed-forward mapping functions while DBN learns a generative model of data. In addition, CNN uses shared weights for all local regions while DBN is a fully connected network (i.e., having different weights for all regions of an image). The training of CNN is entirely discriminative through back-propagation. DBN, on the other hand, employs the layer-wise unsupervised training (e.g., pre-training) followed by the discriminative refinement with back-propagation if necessary. In one embodiment, a CNN is used.

The neural network is defined as a plurality of sequential feature units. Sequential is used to indicate the general flow of output feature values from one unit to input to a next unit. The information from the next layer or unit is fed to a next layer or unit, and so on until the final output. The units may only feed forward or may be bi-directional, including some feedback to a previous unit. The nodes of each unit may connect with all or only a sub-set of nodes of a previous or subsequent unit.

Rather than pre-programming the features and trying to relate the features to attributes, the deep architecture is defined to learn the features at different levels of abstraction. The features are learned to reconstruct lower level features. For example, features for reconstructing an image are learned. For a next unit, features for reconstructing the features of the previous unit are learned, providing more abstraction. Each node of the unit represents a feature. Different units are provided for learning different features.

Within a unit, any number of nodes is provided. For example, 100 nodes are provided. Any number of nodes may be used. A different number of nodes may be provided for different units. Later or subsequent units may have more, fewer, or the same number of nodes. In general, subsequent units have more abstraction. For example, the first unit provides features from the image, such as one node or feature being a line found in the image. The next unit combines lines, so that one of the nodes is a corner. The next unit may combine features (e.g., the corner and length of lines) from a previous unit so that the node provides a shape or building indication. In the example of Figure 2, each box or unit 22, 24, 26 generically represents a plurality of nodes.

The features of the nodes are learned by the machine using any building blocks. For example, auto-encoder (AE) or restricted Boltzmann machine (RBM) approaches are used. AE transforms data linearly, and then applies a non-linear rectification, like a sigmoid function. The objective function of AE is the expected mean square error between the input image and reconstructed images using the learned features. AE may be trained using stochastic gradient descent or other approach to learn, by a machine, the features leading to the best reconstruction.

The objective function of RBM is an energy function. Exact computation of the likelihood term associated with RBM is intractable. Therefore, approximate algorithm, such as contrastive-divergence based on k-step Gibb sampling or other, is used to train the RBM to reconstruct the image from features.

Training of AE or RBM is prone to over-fitting for high-dimensional input data. Sparsity or denoising techniques (e.g., sparse denoising AE (SDAE)) are employed to constrain the freedom of parameters and force learning of interesting structures within the data. Adding noise to training images and requiring the network to reconstruct noise-free images may prevent over-fitting. Enforcing sparsity within hidden layers (i.e., only a small number of units in hidden layers are activated at one time) may also regularize the network. In other embodiments, each or at least one unit is a batch normalization with a ReLU activation followed by a convolution layer (BN+LeakyRU+convolution). Different units may be of the same or different type.

Figure 2 shows one example definition of a network architecture. The network architecture includes an encoder 21 and a decoder 23. The encoder 21 and decoder 23 are formed from various units 22, 24, 26. The network architecture is a dense feature pyramid network formed from the encoder-decoder architecture. The architecture is a fully convolutional network, such that input samples of any size may be used. In alternative embodiments, the architecture is not fully convolutional.

The architecture defines a neural network for deep learning. The architecture is a dense neural network. At least parts of the network include modules or sets 28 of convolutional units 22 that are densely connected. In the example of Figure 2, there are seven sets 28 of densely connected units 22. Other numbers may be provided, such as using only one.

The sets 28 include any number of layers or units 22. Different sets 28 have the same or different numbers of units 22. Each unit 22 includes any number of nodes. The units 22 in a set 28 are arranged in a sequence where the output of a previous unit 22 is used as an input of a subsequent unit 22. For dense connection, the output from each unit 22 is fed directly as an input to all subsequent units 22, not just the immediately subsequent unit 22. Figure 2 shows all subsequent units 22 receiving feature values output from any given unit 22 in the set 28. Each layer or unit 22 of the sequence concatenates output features from all previous ones of the layers or units 22 in the sequence. Each of the convolutional units 22 except the last in sequence in each module 28 includes feed-forward skip connections between the units 22 of the set. In alternative embodiments, output features from less than all the previous units 22 are concatenated. A partially dense connection is provided by having at least one intermediary unit 22 in the sequence receive output features from more than one previous unit 22 in the sequence and/or output features directly to more than one subsequent units 22 in the sequence.

In one embodiment, the sets 28 of units 22 are DenseNet blocks. The feature maps are fed into a 3D DenseNet module 28 with densely connected convolutional blocks 22. Within the DenseNet module 28, the input of each layer 22 comprises the concatenated output features from the previous layers 22. Thus, only a few new features are added to the forwarding information flow together with the identity mappings from the previous layers 22. Various types of layers may be used, such as global average pooling, softmax, and/or sigmoid.

Each convolutional block or unit 22 used in the module 28 contains a batch normalization layer and a ReLu activation followed by a 3x3x3 convolutional layer. Other node arrangements may be used, such as AE and/or RBM.

The architecture is also pyramidal. For example, modules or sets 28 of convolutional blocks or units 22 are separated by down sampling units 24 or up sampling units 26, forming the encoder 21 and decoder 23, respectively. The neural network architecture includes any combination of the sets 28 with down sampling units 24 and up sampling units 26. The down sampling and up sampling units 24, 26 create a pyramid structure of the convolutional blocks or units 22. The pyramid structure corresponds to features at different resolutions. Any number of modules 28, units 22 in a module 28, down sampling units 24, and/or up sampling units 26 may be used. The various units 22, 24, 26 are structured in a pyramidal fashion by use of different resolutions at different stages or parts of the architecture.

Any interconnection between the different units and/or modules may be used. Within the encoder 21, a sequence of modules 28 is provided with decreasing resolution. Each module 28 of the sequence outputs to an input of the next module 28 in the sequence. A down sampling unit 24 is provided between each of the modules or sets 28. Each module 28 operates on features or input data at a different resolution than all, some, or another of the modules 28. In the example of Figure 2, there are 3 DenseNet modules 28 at three different resolutions as the feature encoder 21, combined with 3 down sampling blocks 24. Each module 28 of this example operates at a different resolution than the other modules 28 of the encoder 21, but some modules 28 operating at a same resolution as other modules 28 may be used.

The down sampling blocks 24 employ stride 2 convolution to reduce the feature map sizes. Any level of down sampling may be used, such as down sampling by a factor or stride of 2 (i.e., reducing spatial resolution by V2).

The initial module 28 may operate on the input image data 20 at full resolution. Alternatively and as shown in Figure 2, a down sampling unit 24 down samples prior to the initial module 28. Other intervening units of any type may be provided between any pair of modules 28 or the input medical imaging data 20 and the initial module, or after the final module 28 of the encoder 21. Other sequences through decreasing resolution may be used in the encoder 21.

Within the decoder 23, a sequence of modules 28 is provided with increasing resolution. Each module 28 of the sequence outputs to an input of the next module 28 in the sequence. An up sampling unit 26 is provided between each of the modules or sets 28. Each module 28 operates on features or input data at a different resolution than all, some, or another of the modules 28. In the example of Figure 2, there are 3 DenseNet modules 28 at three different resolutions as the feature decoder 23, combined with 3 up sampling blocks 26. Each module 28 of this example operates at a different resolution than the other modules 28 of the decoder 23, but some modules 28 operating at a same resolution as other modules 28 may be used.

Any level of up sampling may be used, such as up sampling by a factor or stride of 2 (i.e., increasing spatial resolution by ½). The initial module 28 of the decoder 23 may operate on the output data from the encoder 21 at a lowest resolution. The final module 28 of the decoder 23 outputs at a full or initial resolution of the original input medical image data 20. Alternatively and as shown in Figure 2, an up sampling unit 26 up samples after the final module 28 of the decoder 23, providing the output 30. Other intervening units of any type may be provided between any pair of modules 28 or the output heatmap 30 and the final module 28, or before the initial module 28 of the decoder 23. Other sequences through increasing resolution may be used in decoder 23.

The down sampling and up sampling units 24, 26 are three-dimensional convolution layers. The up sampling unit 26 is implemented using the transpose convolution layers of the down sampling unit 24, such as a BN+LeakyRU+Convolution in 3D for down sampling and a BN+LeakyRU+TransposeConvolution in 3D for up sampling. Any size kernel, such as 3x3x3 kernels, may be used. Other types of down sampling and/or up sampling units 24, 26 may be used. The down sampling and up sampling units 24, 26 feed output features into a module 28 or as a final output 30.

The encoder 21 outputs features or values for features to the decoder 23. In the example of Figure 2, another module 28 of densely connected units 22 is provided between the output of the encoder 21 and the input of the decoder 23. The module 28 is the same or different than modules 28 of the encoder 21 and/or decoder 23, such as being a DenseNet module. Given the down sampling unit 24 at the output of the encoder 21 and the transposed up sampler unit 26 at the input of the decoder 23, the in-between module 28 operates on features at a lowest resolution and having the largest effective receptive fields. In other embodiments, this bridging module 28 (and the directly connected down sampling and up sampling units 24, 26) is not provided, is included in the encoder 21, or is included in the decoder 23. Other intervening units may be provided between the encoder 21 and the decoder 23.

Other connections than at the lowest resolution between the encoder 21 and the decoder 23 may be provided. Connections between different parts of the architecture at a same resolution may be used. At each resolution level of the decoder 23, the feature resolution matches the corresponding encoder level. For example, the feature values output from each module 28 or any module 28 in addition to the final module 28 of the encoder 21 are output to the next module 28 in the sequence of the encoder 21 as well as to a module 28 of the decoder 23 with a same resolution. This connection at the same resolution is free of other units or includes other units, such as a down sampling unit 24 and up sampling unit 26 pair in the example of Figure 2. Other connections providing output features as inputs between units 22, 24, 26 and/or modules 28 may be provided. Output at one resolution may be connected to input at a different resolution through additional down sampling and/or up sampling units 24, 26. In alternative embodiments, no other connections than at the lowest resolution are provided between the encoder 21 and the decoder 23.

The decoder 23 up samples the feature maps to the same resolution of the initial encoder 21 resolution level. The output feature map 30 is at a same resolution as the input medical image 20. The output 3D heatmap is obtained by an extra up sampling block 26 with only one output channel. In alternative embodiments, the output feature map 30 is at a different resolution than the input medical image data 20.

Other dense feature pyramidal architectures may be used. Non-dense modules 28 may be provided interspersed with dense modules 28. Partially dense modules 28 may be used. Any number of modules, units, and/or connections may be provided where the operations occur at different resolutions and with at least one module including densely connected units.

In act 44 of Figure 1, a machine (e.g., image processor, workstation, computer or server) trains the neural network arrangement with the training data having ground truth segmentation of the object. The dense feature pyramid neural network is trained using the medical images of the object and the ground truth annotation for the object. Machine learning is performed to train the various units using the defined deep architecture. The features that are determinative or allow reconstruction of inputs are learned. The features providing the desired result or detection of the object are learned.

The results relative to the ground truth and the error for reconstruction for the feature learning network are back-projected to learn the features that work best. In one embodiment, a L2-norm loss is used to optimize the dense feature pyramid network. Other error functions may be used. The optimization is with the Adam algorithm, but other optimization functions may be used. During the optimization, the different distinguishing features are learned. The features providing an indication of location of the object given an input medical image are learned.

In one embodiment, the training data includes 645 patient scans. For each iteration of training, the training batch size is 256. 256 32x32x32 samples are used from the 645 patient scans for a given iteration of training. Multiple iterations are performed. Using the Adam algorithm to optimize with L2-norm error function, the dense pyramid neural network of Figure 2 is optimized with a learning rate of 0.001, beta1=0.9 and beta2=0.999. The optimization takes about 24 hours for 50 training epochs on a 1 Nvidia Titan X Pascal GPU. Other numbers of scans and/or batch sizes may be used. Other sizes of sampling or windows may be used. Other graphics processing units may be used.

The training uses the ground truth data as full segmentations of the object, points of object centroids, or as blobs. For example, Gaussian blobs approximating the object are used. The training creates a machine-learnt detector that outputs estimated locations of Gaussian blobs. Alternatively, the detector learns to output points or full segmentation.

In act 46, the machine outputs a trained neural network. The machine-learnt detector incorporates the deep learned features for the various units and/or modules of the network. The collection of individual features forms a feature or feature set for distinguishing an object from other objects. The features are provided as nodes of the feature units in different levels of abstraction and/or resolutions based on reconstruction of the object from the images. The nodes define convolution kernels trained to extract the features.

Once trained, a matrix is output. The matrix represents the trained architecture. The machine-learnt detector includes definitions of convolution kernels and/or other characteristics of the neural network trained to detect the object of interest, such as lymph nodes. Alternatively, separate matrices are used for any of the nodes, units, modules, network, and/or detector.

The machine-learnt detector is output to a network or memory. For example, the neural network as trained is stored in a memory for transfer and/or later application.

Using the learned features, the machine-learnt detector may detect the object of interest in an input medical image. Once the detector is trained, the detector may be applied. The matrix defining the features is used to extract from an input image. The machine-learnt detector uses the extracted features from the image to detect the object, such as detecting in the form of a spatial distribution or heatmap of likely locations of the object, detecting a full segmentation, and/or detecting a point associated with the object.

Figure 3 is a flow chart diagram of one embodiment of object detection. Figure 3 shows a method for object (e.g., lymph node) detection with a medical imaging system. The machine-learnt detector is applied to detect the object.

The same image processor or a different image processor that used for training applies the learnt features and detector. For example, the matrix or matrices are transmitted from a graphics processing unit used to train to a medical scanner, medical server, or medical workstation. An image processor of the medical device applies the machine-learnt detector. For example, the medical imaging system of Figure 7 is used.

Additional, different, or fewer acts may be provided. For example, acts for scanning a patient and/or configuring the medical system are provided. The acts are performed in the order shown (top to bottom or numerical), but other orders may be used.

In act 54, the image processor receives one or more images of an object. The image is from a scan of a patient and may or may not include the object of interest. For example, CT data represented a volume of a patient (e.g., torso or whole body scan) is received from or by a CT system.

The receipt is by loading from memory. Alternatively, the receipt is by receiving from a network interface. In other embodiments, receipt is by scanning the patient.

The received medical image is to be used to detect whether the object is represented in the image and/or to detect the location or locations of the object or objects of interest. The received medical image may be pre-processed, such as normalized in a same way as the training medical images.

In act 56, the medical imaging system detects whether the input image or part of the image represents the object. For example, the machine-learnt detector determines if one or more lymph nodes are represented in the image. The object is detected using the hidden features of the deep network. For example, the trained convolution units (e.g., BN+LeakyReLU+Convolution units) are applied to the appropriate inputs to extract the corresponding features and output the heatmap. The hidden features are the feature nodes learned at different resolutions. The features of the input image or images are extracted from the image. Other more abstract features may be extracted from those extracted features using the architecture. Depending on the number and/or arrangement of units, other features are extracted from features.

Where the machine-learnt detector is trained based on Gaussian blobs as the segmentation in the training data, the output of the machine-learnt detector may be Gaussian blobs or information derived from Gaussian blobs. Similarly, the detection may find point locations of the object or boundaries of the object.

In one embodiment, the dense feature pyramid neural network is configured by the machine training to output a heatmap at a resolution of the medical image or at another resolution. For example, the neural network outputs a noisy heat-map, o, indicating the likelihood of lymph node presence by location. The locations with the greatest probability (i.e., hottest) are indicated. These locations correspond to detected objects.

The heatmap or other output generated by the machine-learnt detector may be used as the detection. Alternatively, further imaging processing is provided to refine the detection. For example, a machine-trained classifier is applied to the heatmap with or without other input features to refine the detection, such as finding a full segmentation based in part on the heatmap. The machine-trained classifier is trained as part of the optimization of the machine-learnt detector or as a separate optimization.

In another example, further image processing is applied to the output of the neural network as part of the machine-learnt detector. A threshold is applied. The heatmap represents a spatial distribution of probability at each location (e.g., pixel, voxel, or scan sample point) of that location being part of the object. By applying a threshold to this output responsive to input of the medical image to the dense feature pyramid neural network, the locations most likely representing the object are found. Any threshold may be used. For example, *o* is thresholded such that *o*≤ *t* = 0 (where *t* = 0.5). *t* is chosen empirically. Other post processing may be used, such as lowpass filtering the neural network output prior to thresholding, applying cluster analysis instead of or with thresholding, and/or locating the locations of the maximum or X highest locations where X is an integer.

In a further embodiment, the image processor performs non-maximal suppression to results of the application of the threshold. To measure howwell the trained neural network detects each lymph node, the remaining locations clusters in *o* after thresholding are reduced into centroids for matching. Non-maximal suppression is applied such that each cluster is reduced to a single point, given an unknown number of clusters. The neighborhood size for local maxima and matching, *n* and *m*, may have any value. For example, these distances are chosen empirically as *n* = 5 and *m* = 5 pixels or voxels. Skeletonization, region growing, center determination, or other clustering operations may be used.

In act 58, the medical imaging system outputs the detection of the object or objects, such as outputting detection of any lymph nodes. The detection is output. The results or detected information are output. For example, whether there is a match is output. As another example, the probability of match is output. Any information or detection may be output for the object or parts of the object.

In one embodiment, a representation of the medical image with an annotation for the detected object is generated. The output is to an image. The results of the detection indicate whether there is a match or other detection or not. The annotation indicates the location, such as being a marker or graphic for a point, blob, or boarder of the object as detected. In other embodiments, an image of the heatmap is generated.

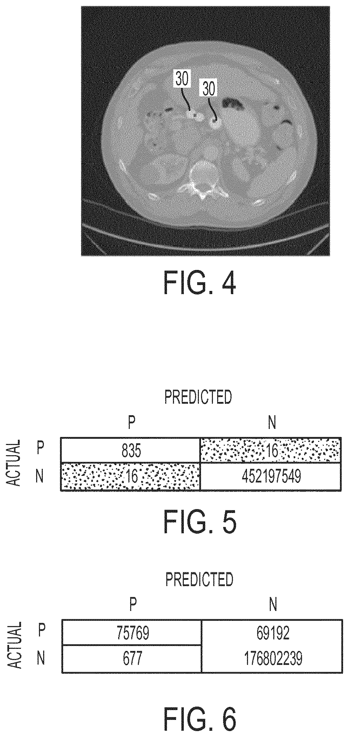

Figure 4 shows an example output as an image of a two-dimensional slice or plane of a scan volume. For explanation, two Gaussian blobs 30 provided in Figure 4 to show the ground truth for training. The dots or points in the blobs 30 are the detected center points of the lymph nodes based on application of the machine-learnt detector and non-maximal suppression with n=5 and m=5. The output for a given patient would be the image with the dots or points highlighted in color or other designation. Alternatively, detected blobs may be highlighted or annotated.

Lymph node detection is a difficult problem. Lymph nodes are small polymorphous structures that resemble vessels and other objects and occur in a variety of backgrounds. Lymph nodes or other objects with similar difficulties may be detected accurately using the trained dense feature pyramid architecture.

The detection for lymph nodes is accurate. For example, 645 patient scans are used for training, and 177 scans are used for evaluation. The dense pyramid neural network architecture as trained performs lymph node detection with 98.1% precision, 98.1% recall, 99.9% specificity, and 99.9% accuracy. This is a significant improvement over previous state-of-the-art of Shin, et al. in "Deep convolutional neural networks for computer-aided detection: Cnn architectures, dataset characteristics and transfer learning," IEEE transactions on medical imaging, vol. 35, no. 5, pp 1285-1298, 2016, which achieves 85% recall and 3 false positives per volume. In contrast, the neural network trained with a dense pyramid architecture of Figure 2 produces 1 false positive for every 11 volumes.

Figure 5 shows the actual and predicted, positive and negative detection of lymph nodes. The machine-learnt detector is trained with Gaussian blobs. Because lymph node centers are a relatively rare item in body scans, the number of negative examples is very large. True negatives are defined by the volume of 3D points that contain neither a true nor predicted lymph node divided by the non-maximal suppression search volume.

Figure 6 shows the actual and predicted, positive and negative detection of lymph nodes using fully annotated segmentation masks instead of Gaussian blobs. The results of using fully annotated segmentation masks yield lymph node detection with precision = 91.1%, recall = 52.2%, specificity = 99.9%, and accuracy = 99.9%. A greater number of false positives results. Using blobs performs better than using masks or actual segmentation.

Detection based on the dense pyramid neural network achieves superior recall and precision scores as compared to a previous lymph node detection algorithm. The neural network architecture combines elements of 3D U-Net (e.g., pyramid) and DenseNet (e.g., densely connected units), along with Gaussian blobs as detection annotations. Physician-assisted diagnosis and treatment of diseases associated with lymph nodes or other objects may be improved, resulting in less review time by physicians.

Figure 7 shows a medical imaging system for object detection, such as detection of lymph nodes in CT scan data. The medical imaging system is a host computer, control station, work station, server, medical diagnostic imaging scanner, or other arrangement used for training and/or application of a machine-learnt detector.

The medical imaging system includes the display 14, memory 16, and image processor 18. The display 14, image processor 18, and memory 16 may be part of the medical CT scanner 11, a computer, server, or other system for image processing medical images from a scan of a patient. A workstation or computer without the CT scanner 11 may be used as the medical imaging system. Additional, different, or fewer components may be provided, such as including a computer network for remote detection of locally captured scans or for local detection from remotely captured scans.

The medical imaging system is for training, such as using images from the memory 16 and/or CT scanner 11 as ground truth. Alternatively, the medical imaging system is for application of the machine-learnt detector trained with the deep dense pyramid network.

The CT scanner 11 is a medical diagnostic CT imaging system. An x-ray source and opposing detector connect with a gantry. The CT scanner 11 is configured to scan a three-dimensional region of the patient 10. The gantry rotates or moves the x-ray source and detector relative to the patient 10, capturing x-ray projections from the source, through the patient 10, and to the detector. Computed tomography is used to generate scan or image data representing the x-ray response of locations distributed in three dimensions within the patient 10. Other medical scanners may be used instead of the CT scanner 11, such as ultrasound, magnetic resonance, positron emission tomography, x-ray, angiography, fluoroscopy, or single photon emission computed tomography.

The image processor 18 is a control processor, general processor, digital signal processor, three-dimensional data processor, graphics processing unit, application specific integrated circuit, field programmable gate array, digital circuit, analog circuit, combinations thereof, or other now known or later developed device for processing medical image data. The image processor 18 is a single device, a plurality of devices, or a network. For more than one device, parallel or sequential division of processing may be used. Different devices making up the image processor 18 may perform different functions, such as an automated anatomy detector and a separate device for generating an image based on the detected object. In one embodiment, the image processor 18 is a control processor or other processor of a medical diagnostic imaging system, such as the CT scanner 11. The image processor 12 operates pursuant to stored instructions, hardware, and/or firmware to perform various acts described herein, such as controlling scanning, detecting an object from scan data, and/or generating an output image showing a detected object.

The image processor 18 is configured to train a deep dense pyramid network. Based on a user provided or other source of the network architecture and training data, the image processor 18 learns features for an encoder and a decoder to train the network. The features are learned at different resolutions. The result of the training is a machine-learnt detector for detecting an object based on the deep dense pyramid architecture. The training data includes samples as Gaussian blobs, points, and/or borders of the object as ground truth, and the learnt detector outputs a corresponding blob, point, and/or border.

Alternatively or additionally, the image processor 18 is configured to detect based on the learned features. The image processor 18 is configured to apply a machine-learnt detector to data from the scan of a patient 10 (i.e., image data from the CT scanner 11). The machine-learnt detector has an architecture including modules of densely connected convolutional blocks, up sampling layers between some of the modules, and down sampling layers between some of the modules. In one embodiment, the architecture of the machine-learnt detector includes one set of the modules arranged in sequence with one of the down sampling layers between each of the modules and includes another set of the modules arranged in sequence with one of the up sampling layers between each of the modules. Any pyramid architecture using down sampling and up sampling may be used. At least one module in the architecture includes densely connected convolution layers or units.

The image processor 18 is configured by application of the machine-learnt detector to output a location (e.g., point, blob, or border) of the object as represented in the data from the scan of a given patient. For example, a heatmap is output. An image of the heatmap shows the distribution of likelihood of the object. The heatmap image may be shown alone or overlaid as color highlighting on an image of the anatomy from the medical image data. The output may be an anatomy image with annotations from further processing of the heatmap or probability detection distribution, such as a point, border, or blob detected by clustering and/or thresholding.

The display 14 is a CRT, LCD, projector, plasma, printer, smart phone or other now known or later developed display device for displaying the output, such as an image with a highlight of a detected object or objects. For example, the display 14 displays a medical image images with an annotation as a marker (e.g., dot or colorization) of the location of the object as detected.

The instructions, medical image, network definition, features, machine-learnt detector, matrices, outputs, and/or other information are stored in a non-transitory computer readable memory, such as the memory 16. The memory 16 is an external storage device, RAM, ROM, database, and/or a local memory (e.g., solid state drive or hard drive). The same or different non-transitory computer readable media may be used for the instructions and other data. The memory 16 may be implemented using a database management system (DBMS) and residing on a memory, such as a hard disk, RAM, or removable media. Alternatively, the memory 16 is internal to the processor 18 (e.g. cache).

The instructions for implementing the object detection in training or application processes, the methods, and/or the techniques discussed herein are provided on non-transitory computer-readable storage media or memories, such as a cache, buffer, RAM, removable media, hard drive or other computer readable storage media (e.g., the memory 16). Computer readable storage media include various types of volatile and nonvolatile storage media. The functions, acts or tasks illustrated in the figures or described herein are executed in response to one or more sets of instructions stored in or on computer readable storage media. The functions, acts or tasks are independent of the particular type of instructions set, storage media, processor or processing strategy and may be performed by software, hardware, integrated circuits, firmware, micro code and the like, operating alone or in combination.

In one embodiment, the instructions are stored on a removable media device for reading by local or remote systems. In other embodiments, the instructions are stored in a remote location for transfer through a computer network. In yet other embodiments, the instructions are stored within a given computer, CPU, GPU or system. Because some of the constituent system components and method steps depicted in the accompanying figures may be implemented in software, the actual connections between the system components (or the process steps) may differ depending upon the manner in which the present embodiments are programmed.

Various improvements described herein may be used together or separately. Although illustrative embodiments of the present invention have been described herein with reference to the accompanying drawings, it is to be understood that the invention is not limited to those precise embodiments, and that various other changes and modifications may be affected therein by one skilled in the art without departing from the scope or spirit of the invention.

## Claims

1. A method for lymph node detection with a medical imaging system, the method comprising:
receiving (54) a medical image of a patient;
detecting (56), by a machine-learnt detector, a lymph node represented in the medical image, the machine-learnt detector comprising a dense feature pyramid neural network of a plurality of groups (28) of densely connected units (22) where the groups (28) are arranged with a first set (21) of the groups (28) connected in sequence with down sampling and a second set (23) of the groups (28) connected in sequence with up sampling and where groups (28) of the first set (21) connect with groups (28) of the second set (23) having a same resolution, and
outputting (58) from the medical imaging system the detection of the lymph node.

2. The method according to claim 1, wherein the medical imaging system comprises a computed tomography (CT) system (11), and wherein receiving (54) the medical image comprises receiving (54) CT data representing a volume of the patient.

3. The method according to any of the preceding claims, wherein detecting (56) by the machine-learnt detector comprises detecting (56) with a fully convolutional network.

4. The method according to any of the preceding claims, wherein detecting (56) comprises detecting (56) with the dense feature pyramid neural network comprising an initial convolutional layer down sampling the medical image,
and/or
wherein detecting (56) comprises detecting (56) with each of the groups (28) comprising a sequence of layers where each layer of the sequence concatenates output features from all previous ones of the layers in the sequence.

5. The method according to any of the preceding claims, wherein detecting (56) comprises detecting (56) with the groups (28) of the first set (21) are in the sequence having the down sampling between each group of the first set (21), each group of the first set (21) having different resolution than the other groups (28) of the first set (21),
and/or
wherein detecting (56) comprises detecting (56) with the groups (28) of the second set (23) are in the sequence having the up sampling between each group of the second set (23), each group of the second set (23) having different resolution than the other groups (28) of the second set (23).

6. The method according to any of the preceding claims, wherein detecting (56) comprises detecting (56) with the first set (21) comprising an encoder and with the second set (23) comprise a decoder,
and/or
wherein detecting (56) comprises detecting (56) with the dense feature pyramid neural network configured to output a heatmap at a resolution of the medical image.

7. The method according to any of the preceding claims, wherein detecting (56) by the machine-learnt detector comprises applying a threshold to an output responsive to input of the medical image to the dense feature pyramid neural network, in particular, wherein detecting (56) by the machine-learnt detector further comprises performing non-maximal suppression to results of the applying of the threshold.

8. The method according to any of the preceding claims, wherein outputting (58) comprises generating a representation of the medical image with an annotation for the lymph node.

9. The method according to any of the preceding claims, wherein detecting (56) by the machine-learnt detector comprises detecting (56) by the machine-learnt detector trained based on Gaussian blobs (30) as segmentation of lymph nodes in training data, and wherein detecting (56) comprises outputting (58) Gaussian blobs (30) for the medical image.

10. A medical imaging system for object detection, the medical imaging system comprising:
a medical scanner (11) configured to scan a three-dimensional region of a patient;
an image processor (18) configured to apply a machine-learnt detector to data from the scan, the machine-learnt detector having an architecture including modules (28) of densely connected convolutional blocks, up sampling layers (26) between some of the modules (28), and down sampling layers (24) between some of the modules (28), the machine-learnt detector configured to output a location of the object as represented in the data from the scan; and
a display (14) configured to display a medical image with an annotation of the object at the location based on the output.

11. The medical imaging system according to claim 10, wherein the medical scanner (11) comprises a computed tomography system, and wherein the image processor (18) and the display are part of the computed tomography system.

12. The medical imaging system according to claims 10 or 11, wherein the architecture of the machine-learnt detector comprises a first set (21) of the modules (28) arranged in sequence with one of the down sampling layers (24) between each of the modules (28) of the first set (21) and a second set (23) of the modules (28) arranged in sequence with one of the up sampling layers (26) between each of the modules (28) of the second set (23).

13. The medical imaging system according to any of the preceding claims 10 to 12, wherein the machine-learnt detector is trained with Gaussian blobs (30) as annotations in the training data and wherein the architecture outputs a heatmap.

14. A method for training for object detection, the method comprising:
defining (42) a neural network arrangement of sets (28) of convolutional blocks (22), the blocks (22) in each set (28) having feed-forward skip connections between the blocks (22) of the set (28), the arrangement including a down sampling layer (24) between a first two of the sets (28) and an up sampling layer (26) between a second two of the sets (28);
training (44), by a machine, the neural network arrangement with training data having ground truth segmentation of the object; and
storing (46) the neural network as trained.

15. The method according to claim 14, wherein defining (42) comprises defining (42) with connections between the first two and the second two of the sets (28) with a same resolution,
and/or
wherein training (44) comprises training (44) with the ground truth segmentation comprising Gaussian blobs (30).
